# EUROPEAN PATENT APPLICATION

(11) **EP 1 808 452 A1**
(43) Date of publication of application: **18.07.2007**
(21) Application number: 05790505.1
(22) Date of filing: 04.10.2005
(51) Int. Cl.: C08G 85/00, A61K 31/787, A61M 1/36, A61P 7/00, A61P 7/08, B01J 20/26, C08F 8/44

(54) **PHOSPHATE ION ADSORBENT**

(30) Priority: 04.10.2004 JP 2004291623
(71) Applicant: Manac Inc., Fukuyama-shi, Hiroshima 721-0956 (JP)
(72) Inventor: KOIKE, Tohru, Hiroshima 732-0063 (JP); TAKEDA, Hironori c/o Minooki Factory, MANAC INC., Hiroshima 721-0956 (JP); SANO, Yoshio c/o Minooki Factory, MANAC INC., Hiroshima 721-0956 (JP); OKADA, Yoshio c/o Minooki Factory, MANAC INC., Hiroshima 721-0956 (JP)
(74) Representative: HOFFMANN EITLE
(86) International application number: PCT/JP2005/018323
(87) International publication number: WO 2006/038603

(57) **Abstract**

The present invention is directed to a phosphate ion adsorbent comprising a polymer or a salt thereof with a metal complex group represented by the following general formula (II): 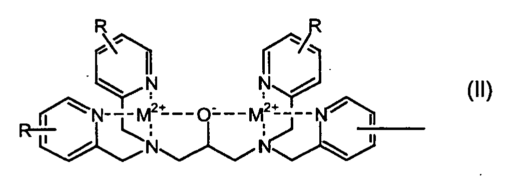 bound thereto directly or through a spacer. The phosphate ion adsorbent of the present invention is advantageous not only in that the adsorbent specifically adsorbs phosphate ions in a living body, but also in that the adsorbent has high adsorbability to phosphate ions, thus useful in removing the phosphate ions from the living body.

## Description

### Field of the Invention

The present invention relates to a polymer or a salt thereof with a specific metal complex group bound thereto, which is advantageous in that the polymer or salt thereof specifically adsorbs phosphate ions in a living body to remove the phosphate ions from the living body, a phosphate ion adsorbent comprising the polymer or salt thereof, a hyperphosphatemia prophylactic and/or therapeutic agent comprising the adsorbent, a blood purification material comprising the adsorbent, a blood purification device using the blood purification material, and a method for adsorbing phosphate ions using the adsorbent.

### Background Art

Hyperphosphatemia is a symptom found in most patients suffering from chronic renal failure and caused by insufficiency of renal function, and, in accordance with the progression to secondary hyperparathyroidism, the patient suffers from various complications. Secondary hyperparathyroidism is believed to be a cause of osteitis fibrosa or ectopic calcification, and recently believed to be a cause of arteriosclerosis, coronary insufficiency, cerebrovascular accidents or the like, and it has been clear that secondary hyperparathyroidism consequently adversely affects vital prognosis. In addition, it is being found that not only secondary hyperparathyroidism but also hyperphosphatemia solely causes complications.

For ameliorating hyperphosphatemia, while employing a dietetic therapy as a basic treatment method, secondary hyperparathyroidism is treated, and further removal of phosphorus by dialysis and administration of an appropriate phosphorus adsorbent are required. Conventionally, as a phosphorus adsorbent, aluminum hydroxide gel or a calcium-based phosphorus adsorbent such as precipitated calcium carbonate has been used. However the aluminum hydroxide gel has a problem in that it causes aluminum osteopathy, aluminum encephalopathy or the like, and therefore the aluminum hydroxide gel is contraindicated in patients needing dialysis, and the calcium-based phosphorus adsorbent poses a problem in that it causes hypercalcemia, ectopic calcification or the like. In recent years, studies are made on the method using an anion-exchange resin as a noncalcium-based phosphorus adsorbent. As examples of anion-exchange resins, there can be mentioned a polymer with a guanidino group bounded thereto (see, for example, International Publication No. WO 94/19379), a polymer obtained by crosslinking polyallylamine hydrochloride with epichlorohydrin (see, for example, International Publication No. WO 95/05184), an anion-exchange resin comprised of a 2-methylimidazole-epichlorohydrin copolymer or a cholestyramine resin (see, for example, Japanese Unexamined Patent Publication No. Hei 9-295941), a weakly basic anion-exchange resin with ferric ions chelated thereto (see, for example, International Publication No. WO 98/03185), a polymer obtained by crosslinking polyethyleneimine with methyl acrylate or epichlorohydrin (see, for example, International Publication Nos. WO 01/66606 and WO 01/66607), a weakly basic anion-exchange resin comprised of an acrylate-divinylbenzene copolymer having a tertiary amino group (see, for example, International Publication No. WO 01/68106), a polymer obtained by crosslinking polylysine with epichlorohydrin (see, for example, Japanese Unexamined Patent Publication No. 2003-33651), and a polymer obtained by crosslinking vinyl monomers having a quaternary ammonium salt with polyethylene glycol methacrylate (see, for example, Japanese Unexamined Patent Publication No. 2003-226648). However, these anion-exchange resins have a problem in that the adsorbability to phosphorus and/or adsorption specificity to phosphate ions is low and hence the amount of the resin used is inevitably increased for obtaining an improved therapeutic effect.

On the other hand, as examples of zinc complexes capable of capturing a substance having a phosphate group, there can be mentioned a zinc complex having a binuclear zinc complex structure crosslinked with an alkoxide, and a polymer carrier with the zinc complex group bound thereto (see, for example, International Publication Nos. WO 03/053932 and WO 04/078828). However, the former, i.e., zinc complex is soluble in a solvent, and therefore, even when the zinc complex captures a substance having a phosphate group in a solvent, it is not easy to remove the substance from the solvent. Further, in the patent document concerning the latter, i.e., polymer carrier with the zinc complex group bound thereto, there is no description showing that the polymer carrier can specifically adsorb phosphate ions in a living body.

### DISCLOSURE OF THE INVENTION

### Problem to be Solved by the Invention

It is an object of the present invention to provide a phosphate ion adsorbent which is advantageous not only in that the adsorbent specifically adsorbs phosphate ions in a living body, but also in that the adsorbent has high adsorbability to phosphate ions, thus useful in removing the phosphate ions from the living body.

### Means to Solve the Problem

The present inventors have conducted extensive and intensive studies with a view toward solving the above-mentioned problems. As a result, it has been found that, when a specific metal complex group is bonded to a polymer or a salt thereof, the resultant polymer or salt thereof has specific and high adsorbability to phosphate ions, and further is hardly soluble (preferably insoluble) in water, or blood or plasma, and therefore the use of the polymer or salt thereof makes very easy the removal of phosphate ions from a living body, namely, the polymer or salt thereof can be a useful phosphate ion adsorbent. Further, a hyperphosphatemia prophylactic and/or therapeutic agent, a blood purification material, a blood purification device, and a method for adsorbing phosphate ions using the adsorbent have also been achieved, and the present invention has been completed.

Specifically, the present invention (1) is directed to a polymer or a salt with a metal complex group represented by the following general formula (I):

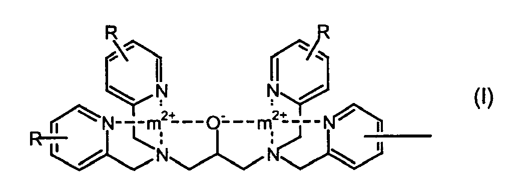

wherein:
m represents a metal atom capable of forming a dication, with the proviso that m is not a zinc atom, and
R each occurrence independently represents a hydrogen atom; an alkyl group having 1 to 16 carbon atoms; an acyl group, an alkoxycarbonyl group, an acylalkyl group, an alkoxycarbonylalkyl group, a carboxyalkyl group, a carbamoylalkyl group, a cyanoalkyl group, a hydroxyalkyl group, an aminoalkyl group, or a haloalkyl group, wherein an alkyl moiety in each group has 1 to 16 carbon atoms; a carboxyl group; a carbamoyl group; a cyano group; a hydroxyl group; an amino group; or a halogeno group,
bound thereto directly or through a spacer.

The present invention (2) is directed to a phosphate ion adsorbent comprising a polymer or a salt thereof with a metal complex group represented by the following general formula (II):

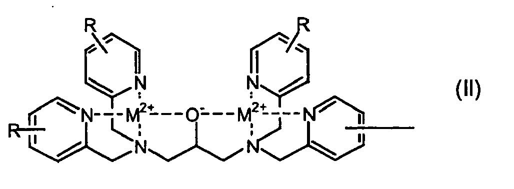

wherein:
M represents a metal atom capable of forming a dication, and
R each occurrence independently represents a hydrogen atom; an alkyl group having 1 to 16 carbon atoms; an acyl group, an alkoxycarbonyl group, an acylalkyl group, an alkoxycarbonylalkyl group, a carboxyalkyl group, a carbamoylalkyl group, a cyanoalkyl group, a hydroxyalkyl group, an aminoalkyl group, or a haloalkyl group, wherein an alkyl moiety in each group has 1 to 16 carbon atoms; a carboxyl group; a carbamoyl group; a cyano group; a hydroxyl group; an amino group; or a halogeno group,
bound thereto directly or through a spacer.

Further, the present invention (3) is directed to a hyperphosphatemia prophylactic and/or therapeutic agent comprising the phosphate ion adsorbent according to the invention (2) above.

The present invention (4) is directed to a blood purification material comprising the phosphate ion adsorbent according to the invention (2) above.

The present invention (5) is directed to a blood purification device using the blood purification material according to the invention (4) above.

Further, the present invention (6) is directed to a method for adsorbing phosphate ions, comprising a step wherein a phosphate ion is adsorbed by being bonded to the phosphate ion adsorbent according to the invention (2) above.

### Effect of the Invention

The polymer or a salt thereof with a metal complex group represented by the general formula (II) bound thereto of the present invention has specific and high adsorbability to phosphate ions, and further is hardly soluble (preferably insoluble) in water, or blood or plasma, and therefore the use of the polymer or salt thereof makes very easy the removal of phosphate ions from a living body. Specifically, the polymer or salt thereof can specifically adsorb phosphate ions, and hence does not substantially affect, e.g., other representative electrolytic components such as chloride ions, carbonate ions or sulfate ions in a living body. In addition, the polymer or salt thereof has excellent adsorbability to phosphate ions, and hence the polymer or salt thereof administered or used in a small amount can exhibit an effect, and thus the amount administered or used can be reduced. Further, the removal of the polymer or salt thereof having adsorbed thereon phosphate ions from water, or blood or plasma can be very easily achieved using a simple operation of, e.g., solid-liquid separation. The polymer exhibits only a phosphate-ion adsorption action and the polymer itself suffers no change, and hence has excellent effect such that it does not cause a side effect, which may be caused by a conventional phosphorus adsorbent, e.g., aluminum hydroxide gel or a calcium-based phosphorus adsorbent, such as precipitated calcium carbonate.

### BRIEF DESCRIPTION OF THE DRAWINGS

Fig. 1 is a graph showing the measurements of anion recovery rates in Examples 5 and 6 and Comparative Example 1.
Fig. 2 is a graph showing the determinations of anion adsorption rates in Examples 5 and 6 and Comparative Example 1.

### BEST MODE FOR CARRYING OUT THE INVENTION

In the metal complex group represented by the general formula (I) or (II) in the present invention, with respect to the "metal atom capable of forming a dication", there is no particular limitation as long as it is a metal atom of typical element or transition element capable of forming a divalent ion having a positive charge. Specific examples of metal atoms include beryllium, magnesium, calcium, titanium, vanadium, chromium, manganese, iron, cobalt, nickel, copper, zinc, germanium, strontium, zirconium, niobium, molybdenum, ruthenium, rhodium, palladium, silver, cadmium, tin, barium, tungsten, rhenium, osmium, iridium, platinum, mercury, lead, polonium, and radium, and preferred examples include magnesium, calcium, chromium, manganese, iron, cobalt, nickel, copper, zinc, and molybdenum, and the most preferred examples include copper and zinc.

In the metal complex group represented by the general formula (I) or (II) in the present invention, the "alkyl group having 1 to 16 carbon atoms" means a linear or branched alkyl group having 1 to 16 carbon atoms.

In the metal complex group represented by the general formula (I) or (II) in the present invention, the "acyl group" means a group R'CO- (wherein R' represents a linear or branched alkyl group having 1 to 16 carbon atoms).

In the metal complex group represented by the general formula (I) or (II) in the present invention, the alkyl moiety and acyl moiety in "an acyl group, an alkoxycarbonyl group, an acylalkyl group, an alkoxycarbonylalkyl group, a carboxyalkyl group, a carbamoylalkyl group, a cyanoalkyl group, a hydroxyalkyl group, an aminoalkyl group, or a haloalkyl group" are as defined above.

Further, with respect to the polymer with the metal complex group represented by the general formula (I) or (II) bound thereto, there is no particular limitation as long as it is a polymer capable of bonding to the above group and serves as a "carrier" or "support". It is preferred that the polymer is comprised of a pharmaceutically acceptable polymer. In addition, it is preferred that the polymer is one which does not adversely affect the phosphate ion adsorption. Specific examples of polymers include polystyrene, polyethylene, polypropylene, polyacetylene (polyyne), polyvinyl chloride, polyvinyl ester, polyvinyl ether, polyvinyl alcohol, polyacrylate, polyacrylic acid, polyacrylonitrile, polyacrylamide, polymethacrylate, polymethacrylic acid, polymethacrylonitrile, polymethacrylamide, polyether, polyacetal, polyester, polyethylene terephthalate, polycarbonate, polyamide, nylon, polyurethane, polyurea, polyimide, polyimidazole, polyoxazole, polysulfide, polysulfone, polysulfonamide, polyether sulfone, polymer alloys, cellulose, cellulose acetate, dextran, dextran sulfate, agarose, chitosan, and silicone, and the polymer may be a copolymer thereof.

The polymer may be a polymer having a crosslinked (bridged) structure, and specific examples of crosslinking agents include divinylbenzene, epichlorohydrin, N,N'-methylenebisacrylamide, and 4,4'-diphenylmethane diisocyanate.

The polymer can be obtained by an appropriate synthesis based on the common general technical knowledge of those skilled in the art, or is commercially available. For example, 4% highly crosslinked agarose is available from Amersham Biosciences Corp. under the trade name of Sepharose (trademark) 4 Fast Flow.

The polymer is bonded to the metal complex group represented by the general formula (I) or (II) directly or through a spacer. The spacer is introduced for making a space between the polymer and the metal complex group to facilitate phosphate ion adsorption of the metal complex group and to increase the degree of swelling. Examples of spacers include a spacer selected from the group consisting of a linear or branched alkylene group or alkenylene group having 1 to 20 carbon atoms (which group is optionally substituted with a carboxyl group, a carbamoyl group, a cyano group, a hydroxyl group, an amino group and/or a halogeno group), -O-, -C(O)-, -N(O)-, -N(R") -, -N⁺(R")₂-, -S(O)ₙ-, and -N=C(R")- (wherein n is an integer of 0 to 2, and R" is hydrogen, a hydroxyl group, or a linear or branched alkyl group having 1 to 6 carbon atoms), and spacers comprised of a combination thereof, but there is no particular limitation as long as the above objective is attained. Specific examples of bonding modes of the metal complex group to the polymer or spacer include covalent bonds, such as a carbon-carbon bond, an ester bond, a carbonyl bond, an amide bond, an ether bond, a sulfide bond, an amino bond, and an imino bond.

The polymer with the metal complex group bound thereto may be present in the form of a salt, and specific examples of salts include inorganic acids, such as hydrochloric acid, sulfuric acid, bicarbonic acid, carbonic acid, and nitric acid; carboxyl group-containing organic acids, such as oxalic acid, tartaric acid, benzoic acid, 4-methoxybenzoic acid, 4-hydroxybenzoic acid, valeric acid, citric acid, glyoxylic acid, glycolic acid, glyceric acid, glutaric acid, chloroacetic acid, chloropropionic acid, cinnamic acid, succinic acid, acetic acid, lactic acid, pyruvic acid, fumaric acid, propionic acid, 3-hydroxypropionic acid, malonic acid, butyric acid, isobutyric acid, amino acids, imidinoacetic acid, malic acid, isethionic acid, citraconic acid, adipic acid, itaconic acid, crotonic acid, salicylic acid, gluconic acid, glucuronic acid, gallic acid, and sorbic acid; and sulfonic acid group-containing organic acids, such as sulfoacetic acid, methanesulfonic acid, and ethanesulfonic acid, preferably pharmaceutically acceptable salts, e.g., halogens; inorganic salts, such as hydrochloride, sulfate, bicarbonate, and carbonate; and organic acids, such as formic acid, acetic acid, propionic acid, malonic acid, succinic acid, fumaric acid, ascorbic acid, glucuronic acid, amino acids, e.g., aspartic acid and glutamic acid, and sulfonic acid.

As a specific example of the polymer or a salt thereof with a metal complex group represented by the general formula (I) bound thereto of the present invention, there can be mentioned a polymer with a copper complex group bound thereto, such as Sepharose (trademark) 4 Fast Flow with a Cu²⁺₂-N,N,N',N'-tetrakis[(2-pyridyl)methyl]-1,3-diamino-2-propoxide group bound thereto through a spacer:

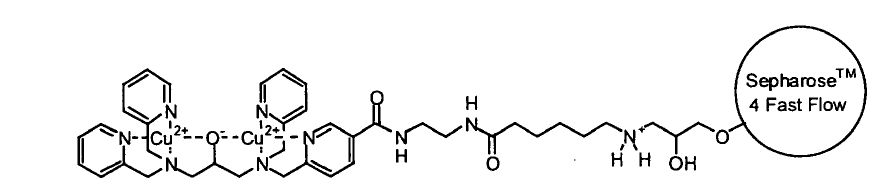

As a specific example of the polymer or a salt thereof with a metal complex group represented by the general formula (II) bound thereto of the present invention, there can be mentioned a polymer with a zinc complex group bound thereto, such as Sepharose (trademark) 4 Fast Flow with a Zn²⁺₂-N,N,N',N'-tetrakis[(2-pyridyl)methyl]-1,3-diamino-2-propoxide group bound thereto through a spacer:

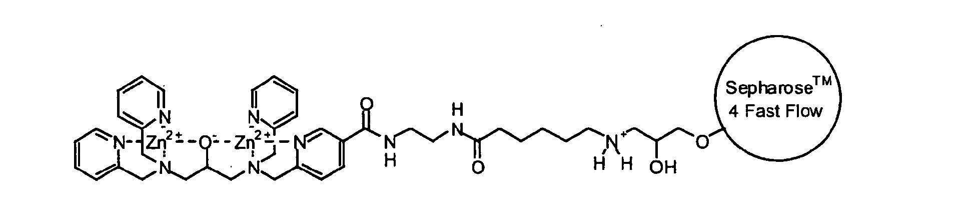

The metal complex group represented by the general formula (II) itself is generally soluble in water, or blood or plasma, but a polymer with the metal complex group bound thereto is hardly soluble (preferably insoluble) in water, or blood or plasma. Therefore, under preferred conditions, the polymer or a salt thereof with a metal complex group represented by the general formula (II) bound thereto of the present invention has specific and high adsorbability to phosphate ions in water, or blood or plasma, and further is hardly soluble (preferably insoluble) in water, or blood or plasma, and hence the removal of the polymer or salt thereof having adsorbed thereon phosphate ions from water, or blood or plasma can be very easily achieved using a simple operation of, e.g., solid-liquid separation. Specifically, the polymer or salt thereof of the present invention has excellent and specific adsorbability to phosphate ions, and hence is extremely useful as a phosphate ion adsorbent.

The phosphate ion adsorbent of the present invention is also extremely useful as a hyperphosphatemia prophylactic and/or therapeutic agent for, e.g., patients suffering from renal failure. Specifically, when the phosphate ion adsorbent comprising a polymer or a salt thereof with a metal complex group represented by the general formula (II) bound thereto is used as a medicament for a patient, the polymer or salt thereof in the medicament passes through the gastrointestinal tract and is finally excreted. During gastrointestinal transit, the polymer or salt thereof adsorbs or captures phosphate ions in the food eaten by the patient to prevent phosphorus absorption or accumulation in the patient body, thus making it possible to reduce the phosphorus concentration of blood. The polymer exhibits only a phosphate-ion adsorption action and the polymer itself suffers no change, and hence does not cause a side effect, which may be caused by a conventional phosphorus adsorbent, e.g., aluminum hydroxide gel or a calcium-based phosphorus adsorbent, such as precipitated calcium carbonate.

As a phosphate ion adsorbent for medical use, particularly as a hyperphosphatemia prophylactic and/or therapeutic agent, the polymer or a salt thereof with a metal complex group represented by the general formula (II) bound thereto, which is an effective ingredient, may be used as it is, but a pharmaceutical composition comprising the polymer or salt thereof as an effective ingredient may be prepared using a general-purpose pharmaceutical additive and formulated by a known method. Examples of dosage forms of the pharmaceutical composition include tablet, capsule, granule, powder, pill, troche, and liquid preparation, and these are applied by oral administration (including sublingual administration).

The pharmaceutical composition for oral administration can be formulated by a conventionally known method, such as mixing, filling, or tabletting. Alternatively, an effective ingredient may be distributed using a replicate mixing operation into the pharmaceutical composition using a great amount of filler. For example, a tablet or capsule for oral administration is preferably provided as a unit dosage form, and may contain a conventional pharmaceutical carrier such as a binder, a filler, a diluent, a tabletting agent, a lubricant, a disintegrant, a colorant, a perfume, a wetting agent, or an enteric coating agent. A tablet may be in the form of a coated tablet prepared in accordance with a known method, for example, using a coating agent (including an enteric coating agent).

Preferred examples of the filler include cellulose, mannitol, lactose and the like. The disintegrant, such as starch, polyvinyl pyrrolidone, or a starch derivative, e.g., sodium starch glycolate; the lubricant, such as sodium laurylsulfate; or the enteric coating agent, such as cellulose acetate phthalate, hydroxypropylmethylcellulose phthalate, hydroxypropylmethylcellulose acetate succinate, carboxymethylethylcellulose, or a methacrylic acid-methyl methacrylate copolymer, can be used as pharmaceutical additives.

The liquid pharmaceutical composition for oral administration is provided in the form of, for example, aqueous or oil suspension, solution, emulsion, syrup, or elixir, or a dried pharmaceutical composition which can be redissolved in water or an appropriate medium before being used. In such a liquid preparation, a known additive, e.g., a precipitation inhibitor, such as sorbitol, syrup, methyl cellulose, gelatin, hydroxyethyl cellulose, carboxymethylcellulose, aluminum stearate gel, or hydrogenated food fat; an emulsifier, such as lecithin, sorbitan monooleate, or gum arabic; an oil ester, such as almond oil, rectified coconut oil, or glycerol ester; a nonaqueous solvent (including food oil), such as propylene glycol or ethyl alcohol; or a preservative, such as methyl 4-hydroxybenzoate, ethyl 4-hydroxybenzoate, propyl 4-hydroxybenzoate, or sorbic acid, and, if necessary, a known a perfume or colorant can be incorporated.

In a preparation comprised of the pharmaceutical composition for oral administration, for example, in a tablet, capsule, granule, or powder, an effective amount of the polymer or a salt thereof with a metal complex group represented by the general formula (II) bound thereto is appropriately determined according to, e.g., the amount of the metal complex group carried and may be contained. The phosphate ion adsorbent for medical use of the present invention is useful in the prevention and/or treatment of hyperphosphatemia due to a disease of renal function impairment, and especially useful in the prevention and/or treatment of hyperphosphatemia accompanying renal function impairment. The dose of the hyperphosphatemia prophylactic and/or therapeutic agent of the present invention may be appropriately determined depending on the age, health condition and weight of a patient, the severity of disease, the type or frequency of medical treatment if the patient is simultaneously subjected to another medical treatment, the nature of desired effect, and others.

The phosphate ion adsorbent of the present invention is also extremely useful as a blood purification material for removing phosphate ions from blood or plasma. Specifically, when the phosphate ion adsorbent comprising a polymer or a salt thereof with a metal complex group represented by the general formula (II) bound thereto is used as a blood purification material in a blood purification method, such as hemodialysis, hemofiltration, hemodiafiltration, hemoadsorption, or plasma adsorption, the polymer or salt thereof in the blood purification material adsorbs or captures phosphate ions in blood or plasma, making it possible to reduce the concentration of phosphorus in blood. The polymer exhibits only a phosphate-ion adsorption action and the polymer itself suffers no change, and therefore the purification material having adsorbed thereon phosphate ions can be disposed of as it is, together with phosphate ions.

As a phosphate ion adsorbent, particularly as a blood purification material, the polymer or a salt thereof with a metal complex group represented by the general formula (II) bound thereto, which is an effective ingredient, may be used as it is. Further the polymer or salt thereof may be copolymerized with or stacked on a material used in a general-purpose blood purification material to form a blood purification material comprising the polymer or salt thereof as an effective ingredient and shape it by a known method. Examples of forms of the blood purification material include hollow fiber membrane, planar membrane, beads, gel, nonwoven fabric and the like.

In the blood purification material comprising the above polymer or salt thereof as an effective ingredient, for example, in a hollow fiber membrane, planar membrane, beads, gel, or nonwoven fabric, an effective amount of the polymer or salt thereof with a metal complex group represented by the general formula (II) bound thereto is appropriately determined according to, e.g., the amount of the metal complex group carried and may be contained. The phosphate ion adsorbent of the present invention is useful in a blood purification therapy due to a disease of renal function impairment, and especially useful in a blood purification therapy accompanying renal function impairment. The form of the blood purification material of the present invention may be appropriately determined depending on a method of treatment employed, e.g., a blood purification therapy.

On setting the blood purification material of the present invention in, e.g., a general-purpose blood purification device, it is extremely useful as a blood purification device for removing phosphate ions from blood or plasma. Examples of such blood purification devices include dialyzers, hemofilters, hemodiafilters, and adsorption-type blood purification devices.

The blood purification device is appropriately used separate depending on the various blood purification methods, which are classified according to a separation technique, such as dialysis, ultrafiltration or adsorption, or the presence or absence of extracorporeal blood circulation, or durability. The phosphate ion adsorbent of the present invention is useful in a blood purification therapy due to a disease of renal function impairment, and especially useful in a blood purification therapy accompanying renal function impairment. The type of the blood purification device of the present invention may be appropriately determined depending on the physique of a patient, the degree of hypercatabolism, and the degree of remaining renal function.

The method for adsorbing phosphate ions of the present invention utilizes a principle that the metal complex group represented by the general formula (II) bonded to the polymer or salt thereof bonds to phosphate ions. The hyperphosphatemia treatment and/or therapeutic agent, blood purification material, or blood purification device is selected according to the method of adsorbing phosphate ions, thus enabling phosphate ion adsorption suitable for each use. The method for adsorbing phosphate ions comprises allowing the phosphate ion adsorbent of the present invention to bind thereto phosphate ions under, e.g., neutral conditions which are physiological conditions.

### Examples

Hereinafter, the present invention will be described in more detail with reference to the following Examples, which should not be construed as limiting the scope of the present invention.

### Example 1

### (Synthesis of ligand)

A solution of 33 g of 1,3-diamino-2-propanol, 116 g of 2-pyridinecarboxaldehyde, and 50 g of sodium cyanotrihydridoborate in 500 mL of a methanol was stirred at room temperature for three days. The resultant solution was worked up, and then purified by column chromatography to yield 34 g of a synthesis intermediate.

A solution of 18 g of the intermediate obtained, 12 g of methyl 6-bromomethylnicotinate, and 14 g of potassium carbonate in 225 mL of N,N-dimethylformamide was stirred at 50°C for one hour. The resultant solution was worked up, and then purified by column chromatography to yield 22 g of a methyl ester derivative.

A solution of 10 g of the methyl ester derivative obtained and 23 g of ethylenediamine in 100 mL of methanol was stirred at room temperature for three days. The resultant solution was worked up, and then purified by column chromatography to yield 10 g of N-{5-[N-(2-amino)ethyl]carbamoyl-2-pyridyl}methyl-N,N',N'-tris[(2-pyridyl)methyl]-1,3-diamino-2-propanol.

### Example 2

### (Synthesis of polymer with a ligand group bound thereto)

10 mL of NHS-activated Sepharose (trademark) 4 Fast Flow was swelled with 10 mL of acetonitrile, and stirred at room temperature for 4 hours, together with 10 mL of a 4.0 mM solution of the N-{5-[N-(2-amino)ethyl]carbamoyl-2-pyridyl}methyl-N,N',N'-tris[(2-pyridyl)methyl]-1,3-diamino-2-propanol obtained in Example 1 in acetonitrile. The resultant reaction mixture was filtered and washed, followed by washing with a mixed solution comprising a 0.1 M aqueous solution of sodium hydrogencarbonate and a 0.1 M aqueous solution of sodium carbonate, to yield 10 mL of Sepharose (trademark) 4 Fast Flow with an N,N,N',N'-tetrakis[(2-pyridyl)methyl]-1,3-diamino-2-propanol group bound thereto through a spacer.

### Example 3

### (Synthesis of polymer with a copper complex group bound thereto)

0.1 mL of Sepharose (trademark) 4 Fast Flow with an N,N,N',N'-tetrakis[(2-pyridyl)methyl]-1,3-diamino-2-propanol group bound thereto through a spacer obtained in Example 2 and 0.2 mL of a mixed solution comprising a 0.1 M 2-(N-morpholino)ethanesulfonic acid (MES) buffer (pH 6.0) and a 0.1 M aqueous solution of copper acetate were stirred at room temperature for one hour. The resultant reaction mixture was filtered and washed, followed by washing with a 20 mM Tris-acetic acid buffer (pH 7.4), to yield 0.1 mL of Sepharose (trademark) 4 Fast Flow with a Cu²⁺₂-N,N,N' ,N'-tetrakis[(2-pyridyl)methyl]-1,3-diamino-2-propoxide group bound thereto through a spacer.

### Example 4

### (Synthesis of polymer with a zinc complex group bound thereto)

0.1 mL of Sepharose (trademark) 4 Fast Flow with an N,N,N',N'-tetrakis[(2-pyridyl)methyl]-1,3-diamino-2-propanol group bound thereto through a spacer obtained in Example 2 and 0.2 mL of a mixed solution comprising a 0.1 M 2-(N-morpholino)ethanesulfonic acid (MES) buffer (pH 6.0) and a 0.1 M aqueous solution of zinc acetate were stirred at room temperature for one hour. The resultant reaction mixture was filtered and washed, followed by washing with a 20 mM Tris-acetic acid buffer (pH 7.4), to yield 0.1 mL of Sepharose (trademark) 4 Fast Flow with a Zn²⁺₂-N,N,N',N'-tetrakis[(2-pyridyl)methyl]-1,3-diamino-2-propoxide group bound thereto through a spacer.

### Example 5

### (Measurement of anion adsorption of polymer with a copper complex group bound thereto)

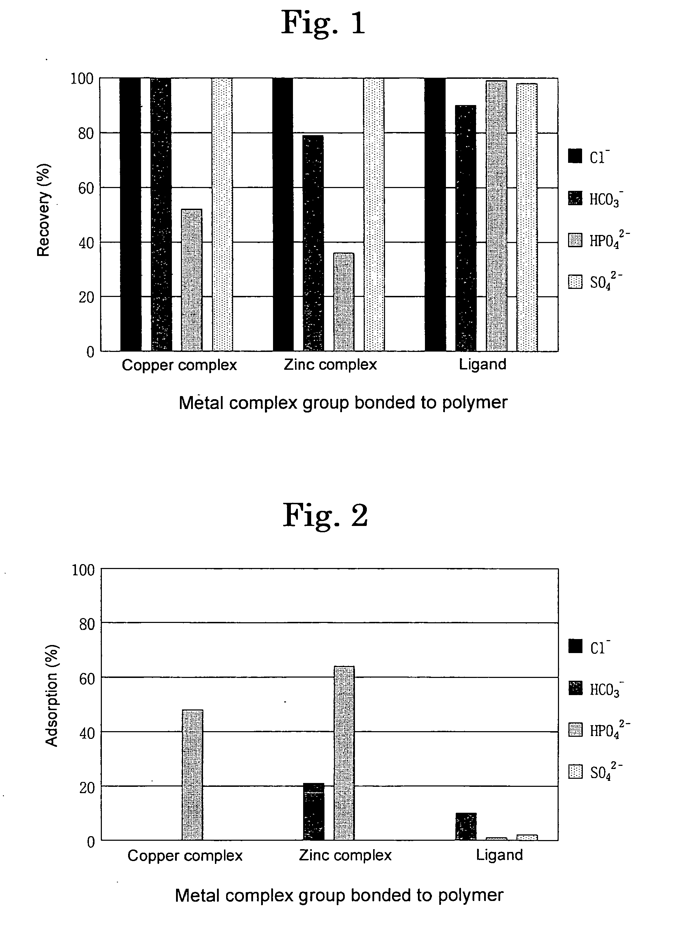

0.1 mL of Sepharose (trademark) 4 Fast Flow with a Cu²⁺₂-N,N,N',N'-tetrakis[(2-pyridyl)methyl]-1,3-diamino-2-propoxide group bound thereto through a spacer obtained in Example 3 was washed with ultrapure water, and then 0.1 mL of a mixed aqueous solution (pH 7.4) containing 103 mM Cl⁻, 27.0 mM HCO₃⁻, 2.26 mM HPO₄²⁻, and 0.500 mM SO₄²⁻ was added, followed by shaking at 37°C for 5 minutes. The resultant mixed aqueous solution was filtered, and the filtrate was collected and then washed with 0.1 mL of ultrapure water 9 times, and all of the filtrates was collected. A recovery rate of each anion in the filtrate collected was measured using ion chromatography. As a result, there were obtained the following measurements: Cl⁻: 100%, HCO₃⁻: 100%, HPO₄²⁻: 52%, SO₄²⁻: 100%. The measurements of anion recovery rates are shown in Fig. 1. Further, from the anion recovery rates measured, an adsorption rate of each anion for Sepharose (trademark) 4 Fast Flow with a Cu²⁺₂-N,N,N',N'-tetrakis[(2-pyridyl)methyl]-1,3-diamino-2-propoxide group bound thereto through a spacer was determined. As a result, there were obtained the following measurements: Cl⁻: 0%, HCO₃⁻: 0%, HPO₄²⁻: 48%, SO₄²⁻: 0%. The determinations of anion adsorption rates are shown in Fig. 2.

### Example 6

### (Measurement of anion adsorption rate of polymer with a zinc complex group bound thereto)

0.1 mL of Sepharose (trademark) 4 Fast Flow with a Zn²⁺₂-N,N,N',N'-tetrakis[(2-pyridyl)methyl]-1,3-diamino-2-propoxide group bound thereto through a spacer obtained in Example 4 was washed with ultrapure water, and then 0.1 mL of a mixed aqueous solution (pH 7.4) containing 103 mM Cl⁻, 27.0 mM HCO₃⁻, 2.26 mM HPO₄²⁻, and 0.500 mM SO₄²⁻ was added, followed by shaking at 37°C for 5 minutes. The resultant mixed aqueous solution was filtered, and the filtrate was collected and then washed with 0.1 mL of ultrapure water 9 times, and all of the filtrates was collected. A recovery rate of each anion in the filtrate collected was measured using ion chromatography. As a result, there were obtained the following measurements: Cl⁻: 100%, HCO₃⁻: 79%, HPO₄²⁻: 36%, SO₄²⁻: 100%. The measurements of anion recovery rates are shown in Fig. 1. Further, from the anion recovery rates measured, an adsorption rate of each anion for Sepharose (trademark) 4 Fast Flow with a Zn²⁺₂-N,N,N',N'-tetrakis[(2-pyridyl)methyl]-1,3-diamino-2-propoxide group bound thereto through a spacer was determined. As a result, there were obtained the following measurements: Cl⁻: 0%, HCO₃⁻: 21%, HPO₄²⁻: 64%, SO₄²⁻: 0%. The determinations of anion adsorption rates are shown in Fig. 2.

### Example 7

### (Measurement of serum phosphate-ion adsorption of polymer with a zinc complex group bound thereto)

0.4 mL of Sepharose (trademark) 4 Fast Flow with a Zn²⁺2-N,N,N',N'-tetrakis[(2-pyridyl)methyl]-1,3-diamino-2-propoxide group bound thereto through a spacer obtained in Example 4 was washed with ultrapure water, and then 0.4 mL of fetal bovine serum (4.0 mM HPO₄²⁻) was added, followed by shaking at 37°C for 5 minutes. The resultant fetal bovine serum was filtered, and the filtrate was collected. The filtrate collected was used as a test liquid, and subjected to deproteinization using trichloroacetic acid, and an aqueous solution of ammonium molybdate in sulfuric acid was added to the test liquid to form molybdophosphoric acid in the test liquid. The resultant liquid was reduced with 1-amino-2-naphthol-4-sulfonic acid, and the molybdenum blue formed was quantitatively determined by colorimetry using an ultraviolet-visible spectrophotometer. As a result, it was found that a phosphate ion (HPO₄²⁻) concentration in the test liquid was 1.5 mM. That is, an amount of a serum phosphate-ion adsorption of Sepharose (trademark) 4 Fast Flow with a Zn²⁺₂-N,N,N',N'-tetrakis[(2-pyridyl)methyl]-1,3-diamino-2-propoxide group bound thereto through a spacer was 2.5 mM.

### Comparative Example 1

### (Measurement of anion adsorption rate of polymer with a ligand group bound thereto)

0.1 mL of Sepharose (trademark) 4 Fast Flow with an N,N,N' ,N'-tetrakis[(2-pyridyl)methyl]-1,3-diamino-2-propanol group bound thereto through a spacer obtained in Example 2 was washed with ultrapure water, and then 0.1 mL of a mixed aqueous solution (pH 7.4) containing 103 mM Cl⁻, 27.0 mM HCO₃⁻, 2.26 mM HPO₄²⁻, and 0.500 mM SO₄²⁻ was added, followed by shaking at 37°C for 5 minutes. The resultant mixed aqueous solution was filtered, and the filtrate was collected and then washed with 0.1 mL of ultrapure water 9 times, and all of the filtrates was collected. A recovery rate of each anion in the filtrate collected was measured using ion chromatography. As a result, there were obtained the following measurements: Cl⁻: 100%, HCO₃⁻: 90%, HPO₉²⁻: 99%, SO₄²⁻ : 98%. The measurements of anion recovery rates are shown in Fig. 1. Further, from the anion recovery rates measured, an adsorption rate of each anion for Sepharose (trademark) 4 Fast Flow with an N,N,N',N'-tetrakis[(2-pyridyl)methyl]-1,3-diamino-2-propanol group bound thereto through a spacer was determined. As a result, there were obtained the following measurements: Cl⁻: 0%, HCO₃⁻: 10%, HPO₄² : 1%, SO₄²⁻: 2%. The determinations of anion adsorption rates are shown in Fig. 2.

### INDUSTRIAL APPLICABILITY

The phosphate ion adsorbent comprising a polymer or a salt thereof with a metal complex group bound thereto of the present invention has specific and high adsorbability to phosphate ions, and further is hardly soluble (preferably insoluble) in water, or blood or plasma, and therefore the use of the adsorbent makes very easy the removal of phosphate ions from a living body, and hence the phosphate ion adsorbent is advantageously used in the prevention and/or treatment of hyperphosphatemia due to a disease of renal function impairment or in the blood purification method.

## Claims

1. A polymer or a salt thereof with a metal complex group represented by the following general formula (I): 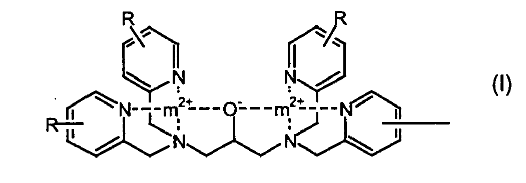 wherein:
m represents a metal atom capable of forming a dication, with the proviso that m is not a zinc atom, and
R each occurrence independently represents a hydrogen atom; an alkyl group having 1 to 16 carbon atoms; an acyl group, an alkoxycarbonyl group, an acylalkyl group, an alkoxycarbonylalkyl group, a carboxyalkyl group, a carbamoylalkyl group, a cyanoalkyl group, a hydroxyalkyl group, an aminoalkyl group, or a haloalkyl group, wherein an alkyl moiety in each group has 1 to 16 carbon atoms; a carboxyl group; a carbamoyl group; a cyano group; a hydroxyl group; an amino group; or a halogeno group,
bound thereto directly or through a spacer.

2. A phosphate ion adsorbent comprising a polymer or a salt thereof with a metal complex group represented by the following general formula (II): 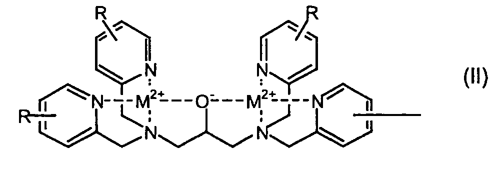 wherein:
M represents a metal atom capable of forming a dication, and
R each occurrence independently represents a hydrogen atom; an alkyl group having 1 to 16 carbon atoms; an acyl group, an alkoxycarbonyl group, an acylalkyl group, an alkoxycarbonylalkyl group, a carboxyalkyl group, a carbamoylalkyl group, a cyanoalkyl group, a hydroxyalkyl group, an aminoalkyl group, or a haloalkyl group, wherein an alkyl moiety in each group has 1 to 16 carbon atoms; a carboxyl group; a carbamoyl group; a cyano group; a hydroxyl group; an amino group; or a halogeno group,
bound thereto directly or through a spacer.

3. A hyperphosphatemia prophylactic and/or therapeutic agent comprising the phosphate ion adsorbent according to claim 2.

4. A blood purification material comprising the phosphate ion adsorbent according to claim 2.

5. A blood purification device using the blood purification material according to claim 4.

6. A method for adsorbing phosphate ions, comprising a step wherein a phosphate ion is adsorbed by being bonded to the phosphate ion adsorbent according to claim 2.
